# EUROPEAN PATENT APPLICATION

(11) **EP 4 809 964 A1**
(43) Date of publication of application: **23.09.2026**
(21) Application number: 24891211.5
(22) Date of filing: 29.10.2024
(51) Int. Cl.: A61B 90/90, A61C 7/08

(54) **SURGICAL INSTRUMENT MANAGEMENT METHOD AND DEVICE, AND ORTHODONTIC MOUTHPIECE MANAGEMENT METHOD AND DEVICE**

(30) Priority: 16.11.2023 JP 2023194876
(71) Applicant: Shimada, Katsumi, Kasukabe-shi, Saitama 344-0032 (JP)
(72) Inventor: Shimada, Katsumi, Kasukabe-shi, Saitama 344-0032 (JP)
(74) Representative: Schön, Christoph
(86) International application number: PCT/JP2024/038565
(87) International publication number: WO 2025/105170

(57) **Abstract**

It is an object of the present invention to provide a surgical instrument management method and apparatus capable of accurately performing individual identification and management of surgical instruments easily and at low cost. The surgical instrument management method for managing surgical instruments, comprising: a step of obtaining dimensions of specific portions that are predefined for a plurality of surgical instruments; a step of identifying the surgical instruments based on the obtained dimensions of the specific portions; and a step of managing the identified surgical instruments.

## Description

### TECHNICAL FIELD

The present invention relates to a method and apparatus for managing surgical instruments, as well as a method and apparatus for managing orthodontic mouthpieces.

### BACKGROUND ART

An outbreak of Creutzfeldt-Jakob disease (CJD) that became prominent in the 1990s renewed awareness of the importance of individual management of the surgical instruments (that is, tracking which specific instrument was used, when, on which patient, and in which surgical procedure). In 2006, the Ministry of Health, Labour and Welfare issued a notice requiring individual management of the steel instruments (steel surgical instruments). Furthermore, the revision of the Medical Care Act in April 2007 mandated evidence-based operation and management regarding the service life, cost, number of uses, and frequency of use of the steel instruments to ensure their safe use. Although the surgical instruments are commonly made of metals such as stainless steel or titanium and are therefore referred to as steel instruments, in recent years, the surgical instruments made from non-metallic materials such as engineering plastics and ceramics have also been increasing.

Additionally, in 2014, the U.S. FDA legislated the requirement for all surgical instruments to bear a Unique Device Identification (UDI), mandating that the UDI be applied to all surgical instruments by 2020. Similar requirements were legislated in Europe in 2017. However, not only in Japan but globally, individual tracking of the surgical instruments is often not implemented.

Therefore, various methods have been tried and tested over time to address the need for individual identification and management of the surgical instruments used in medical procedures. One known method involves using two-dimensional codes to identify and manage the surgical instruments (Non-Patent Document 1). Another known method uses RFID (Radio Frequency Identification) tags to identify and manage the surgical instruments (Patent Documents 1 to 3 and Non-Patent Document 2).

### PRIOR ART DOCUMENTS

### [Parent Document]

Patent Document 1: JP 2013-033370 A
Patent Document 2: JP 2014-174647 A
Patent Document 3: WO 2021/075159 A1

### [Non-Patent Document]

Non-Patent Document 1: Japanese Society of Medical Instrumentation, Report on the Study of the Necessity of Two-Dimensional Symbol Marking on Steel Surgical Instruments for Surgical Procedures, March 2007
Non-Patent Document 2: Tsutomu Sawa, Individual Management System for Steel Instruments Using RFID: Case Study at Shimane University Hospital, June 2019

### SUMMARY OF INVENTION

### [Technical Problem]

However, the method of identifying and managing the surgical instruments using the aforementioned two-dimensional codes (such as in Non-Patent Document 1) has the following problems. First, since the surgical instruments are used repeatedly over many years through cycles of surgery, disinfection, washing and sterilization, there is a problem where the two-dimensional codes deteriorate during these processes, making it impossible to read the codes. In addition, while the two-dimensional codes are laser-engraved onto the surgical instruments, since the surgical instruments generally cannot be taken out of the hospital, there is a problem that a laser-engraving machine must be brought into the hospital and the engraving must be performed on-site. In this case, because the two-dimensional codes are extremely small, measuring approximately 3 mm square (for example, about 3.6 mm × 1.2 mm), special techniques are required to laser-engrave them onto the surgical instruments. Furthermore, because there are a vast number of types of surgical instruments (numbering in the thousands), expert knowledge is required to associate each two-dimensional code with its corresponding instrument.

Thus, laser-engraving two-dimensional codes onto the surgical instruments presents the problem that both the engraving process itself and the task of linking each two-dimensional code with its corresponding instrument require substantial cost and effort. In addition, since some surgical instruments are made of materials on which the two-dimensional codes cannot be engraved, or are so small that there is no available space for engraving, there is also the problem that it is not possible to engrave the two-dimensional codes on all surgical instruments, and therefore only some of the surgical instruments can be individually managed.

Due to the above problems, the method of identifying and managing the surgical instruments using the two-dimensional codes has not become widespread.

On the other hand, although there is the method for identifying and managing the surgical instruments using the above-mentioned RFID tags (e.g., Non-Patent Document 2), such as attaching the RFID tags operating in the HF band (13.56 MHz) to the surgical instruments, this method has the following problems. The surgical instruments are reused repeatedly through the processes such as surgery, disinfection, washing and sterilization. When the RFID tag is attached to the surgical instrument solely with adhesive, the adhesive strength deteriorates during washing and sterilization processes, causing the RFID tag to detach from the instrument. Therefore, a method has been employed in which a ceramic RFID tag is placed inside a stainless steel housing, and the stainless steel housing is laser-welded to the surgical instrument. After placing the RFID tag in the stainless steel housing, the RFID tag is covered with a special engineering plastic to eliminate gaps and improve washability. This is highly costly and labor-intensive. Additionally, to laser-weld the surgical instrument to the flat surface of the stainless steel housing, it is necessary to perform machining to grind the surgical instrument prior to laser welding.

For these reasons, attaching the RFID tags to the surgical instruments requires a considerable amount of time. Non-Patent Document 2 reports that attaching the RFID tags to 19,584 surgical instruments took 2.5 months. In addition, the welding of the surgical instrument to the stainless steel housing must be performed manually, presenting the further problem that it is difficult to maintain uniform welding quality.

Furthermore, in the method in which the ceramic RFID tag is placed inside the stainless steel housing and the housing is laser-welded to the surgical instrument, the welding must be performed between metal surfaces that each have a certain area. As a result, the locations where the RFID tag can be attached are limited. In addition, because the RFID tags used in this method cannot be used directly on metal surfaces, they protrude from the surgical instrument, creating the risk of interfering with surgical procedures. Even the smallest RFID tags-for example, those measuring 5.2 mm in diameter and 2.0 mm in thickness-cause the stainless steel housing containing the tag to protrude by as much as 6.5 mm from the surgical instrument. Especially, since metal-compatible tags attached to metal surfaces are even larger at 11 mm × 10 mm × 3.5 mm, the required mounting area becomes larger, and the tags protrude even further from the surgical instrument.

In addition, there is the problem that external impacts applied directly to the stainless steel housing or the RFID tag can damage the RFID tag, and there is also the problem that defects in the laser welding can cause the RFID tag to detach. Furthermore, when the surgical instrument is made of materials such as engineering plastic or ceramic, the ceramic RFID tag placed inside the stainless steel housing cannot be laser-welded to the surgical instrument, which presents another problem.

Due to the above problems, the method of identifying and managing the surgical instruments using the RFID tags has not become widespread.

For these reasons, although attempts such as engraving the two-dimensional codes and attaching the RFID tags have been made since the individual management of the surgical instruments became legally mandated in Japan in 2007, the individual management has still not been achieved in most hospitals-except for a very small number-despite about twenty years having passed since discussions began and sixteen years since legalization, due to the various problems described above.

By the way, while traditional orthodontic techniques used wire-based braces, this method had the problem of being unattractive because the metal wires were visible. For this reason, in recent years, orthodontic techniques using transparent, less noticeable mouthpieces (orthodontic mouthpieces) have emerged and rapidly become popular. These orthodontic mouthpieces are produced in a plurality of sets for the same patient, each corresponding to a different stage of the treatment. It is desirable to accurately identify and manage each individual mouthpiece, but because they are transparent, distinguishing the plurality of mouthpieces for the same patient is difficult. Moreover, for reasons similar to those described above for the surgical instruments, it is also difficult to identify and manage them using conventional methods such as the two-dimensional codes or the RFID tags.

The present invention has been made based on the above knowledge. It is an object of the present invention to provide a surgical instrument management method and apparatus capable of accurately performing individual identification and management of surgical instruments easily and at low cost, as well as an orthodontic mouthpiece management method and apparatus capable of accurately performing individual identification and management of orthodontic mouthpieces easily and at low cost.

### [Solution to Technical Problem]

In order to achieve the above object, the present invention provides a surgical instrument management method for managing surgical instruments, comprising: a step of obtaining dimensions of specific portions that are predefined for a plurality of surgical instruments; a step of identifying the surgical instruments based on the obtained dimensions of the specific portions; and a step of managing the identified surgical instruments.

The above present invention identifies the plurality of surgical instruments based on the dimensions of the predetermined specific portions in which individual differences appear among the plurality of surgical instruments (in other words, the dimensions that allow the plurality of surgical instruments to be distinguished from one another). As a result, the identification and management of the surgical instruments can be performed easily, accurately, and at low cost. That is, according to the present invention, it is possible to identify and manage the surgical instruments without encountering the problems associated with the methods that use the two-dimensional codes or the RFID tags to individually identify and manage surgical instruments, as discussed in the section "Technical Problem."

Preferably, in the present invention, the step of obtaining comprises, when the dimensions of the specific portion obtained from a plurality of the surgical instruments for which the same specific portion is defined are identical, newly obtaining one or more dimensions other than the dimensions of the specific portion, from the plurality of the surgical instruments, and the step of identifying comprises identifying the plurality of surgical instruments based on the one or more dimensions.

According to the present invention, even when the dimensions of the specific portion obtained from the plurality of surgical instruments are identical, the plurality of surgical instruments can be accurately identified based on the one or more dimensions other than the dimensions of the specific portion.

Preferably, in the present invention, the step of obtaining comprises, when the dimensions of the specific portion obtained from a plurality of the surgical instruments in which the same specific portion is defined are identical, newly obtaining the dimensions of the specific portion after trimming (shaving) for one or more of the plurality of surgical instruments, and the step of identifying comprises identifying the plurality of surgical instruments based on the dimensions of the specific portion after trimming (shaving).

According to the present invention, even when the dimensions of the specific portion obtained from the plurality of the surgical instruments are identical, the plurality of the surgical instruments can be accurately identified based on the dimensions of the specific portion after trimming (shaving).

Preferably, in the present invention, the surgical instrument management method further comprises a step of determining that, when the dimensions of the specific portion obtained from the plurality of the surgical instruments fall within a predetermined tolerance, the dimensions of the specific portion are identical.

According to the present invention, when the obtained dimensions of the specific portion fall within the predetermined tolerance, these dimensions can be regarded as identical.

Preferably, in the present invention, the step of obtaining comprises obtaining the dimensions of the specific portions of a plurality of the surgical instruments in the same step of obtaining, and the step of identifying comprises identifying the plurality of surgical instruments based on the dimensions of the specific portions that have been obtained in the same step of obtaining.

According to the present invention, the identification of the plurality of the surgical instruments can be performed efficiently.

Preferably, in the present invention, the surgical instrument management method further comprises a step of storing the dimensions of the specific portions obtained from a plurality of the surgical instruments in advance, wherein the step of identifying comprises comparing the dimensions of the specific portions stored in the step of storing with the dimensions of the specific portions obtained in the step of obtaining after the step of storing, to identify the surgical instruments having the dimensions of the specific portions obtained in the step of obtaining.

According to the present invention, the surgical instruments can be accurately identified by performing the comparison with the previously stored dimensions of the specific portions.

In a preferred example of the present invention, the dimension of the specific portion is a width of a protruding portion of the surgical instrument. In another preferred example, the dimension of the specific portion is a width of an end portion of the surgical instrument. In still another preferred example, the dimension of the specific portion is a width of a portion of the surgical instrument that passes through a center of a pivot shaft of the surgical instrument.

Preferably, in the present invention, the step of identifying comprises identifying the surgical instruments based on numerical values in micron or submicron units of the dimensions of the specific portions.

According to the present invention, since the dimensions in micron or submicron units are used, the surgical instruments can be identified with higher accuracy.

Preferably, in the present invention, the step of identifying and the step of managing each comprise using numerical values corresponding to the dimensions of the specific portions, as IDs, in order to identify and manage the surgical instruments.

According to the present invention, the identification and management of the surgical instruments can be performed easily and accurately.

Preferably, in the present invention, the step of managing comprises managing, in association with the IDs, one or more of a name of the surgical instrument, a dimension of the specific portion of the surgical instrument, a group name of the surgical instrument, a subcategory of the surgical instrument, a manufacturer name of the surgical instrument, a usage history of the surgical instrument, a service life of the surgical instrument, a repair history of the surgical instrument, and a purchase history of the surgical instrument.

Preferably, in the present invention, the surgical instrument management method further comprises a step of obtaining respective heights of a plurality of the surgical instruments, and wherein the step of identifying comprises identifying the surgical instruments based on both the obtained heights and the dimensions of the specific portions.

According to the present invention, the surgical instruments can be identified more accurately.

Preferably, in the present invention, the surgical instrument management method further comprises a step of obtaining one or more of a surface roughness of the surgical instrument, a surface gloss of the surgical instrument, a type of an element contained in a material constituting the surgical instrument, and a composition ratio of the element, and wherein the step of identifying comprises identifying the surgical instruments based on one or more of the obtained surface roughness, surface gloss, type of the element, and composition ratio, in addition to the dimensions of the specific portions.

According to the present invention, the surgical instruments can be identified more accurately.

Preferably, in the present invention, the step of managing comprises managing the surgical instruments in sterilization-container units including a plurality of the surgical instruments.

According to the present invention, the plurality of surgical instruments can be managed easily and accurately.

According to another aspect, in order to achieve the above object, the present invention provides a surgical instrument management apparatus for managing surgical instruments, comprising: an obtaining unit configured to obtain dimensions of specific portions that are predefined for a plurality of surgical instruments; an identifying unit configured to identify the surgical instruments based on the obtained dimensions of the specific portions; and a managing unit configured to manage the identified surgical instruments.

In still another aspect, the present invention provides an orthodontic mouthpiece management method, comprising: a step of obtaining dimensions of specific portions that are predefined for a plurality of orthodontic mouthpieces; a step of identifying the orthodontic mouthpieces based on the obtained dimensions of the specific portions; and a step of managing the identified orthodontic mouthpieces.

In yet another aspect, the present invention provides an orthodontic mouthpiece management apparatus, comprising: an obtaining unit configured to obtain dimensions of specific portions that are predefined for a plurality of orthodontic mouthpiece; an identifying unit configured to identify the orthodontic mouthpiece based on the obtained dimensions of the specific portions; and a managing unit configured to manage the identified orthodontic mouthpiece.

### [Effect of Invention]

According to the present invention, a surgical instrument management method and apparatus can accurately perform individual identification and management of surgical instruments easily and at low cost, and an orthodontic mouthpiece management method and apparatus can accurately perform individual identification and management of orthodontic mouthpieces easily and at low cost.

### BRIEF DESCRIPTION OF DRAWINGS

FIG. 1 is a block diagram showing an outline configuration of a surgical instrument management system to which a surgical instrument management method and apparatus according to an embodiment of the present invention are applied.
FIG. 2 shows an overall view of forceps in an open state and an enlarged view of a ratchet of the forceps.
FIG. 3 shows a further enlarged view of the ratchet of the forceps.
FIG. 4 shows an enlarged view of grooves formed in the ratchet of the forceps.
FIG. 5 shows an overall view of scissors in a closed state and an enlarged view of a pivot shaft of the scissors.
FIG. 6 shows an overall view of a needle holder in a closed state and an enlarged view of a tip of a gripping portion of the needle holder.
FIG. 7 shows an overall view of tweezers and an enlarged view of a rear end of a connecting portion of the tweezers.
FIG. 8 is a flowchart showing master registration of surgical instruments performed by a surgical instrument management apparatus in an embodiment of the present invention.
FIG. 9 is a flowchart showing individual identification and management of surgical instruments performed by a surgical instrument management apparatus in an embodiment of the present invention.
FIG. 10 is a plan view of an orthodontic mouthpiece used in a modification of an embodiment of the present invention.

### DESCRIPTION OF EMBODIMENTS

With reference to the accompanying drawings, a surgical instrument management method and apparatus according to an embodiment of the present invention will now be described.

### [Configuration of Surgical Instrument Management System]

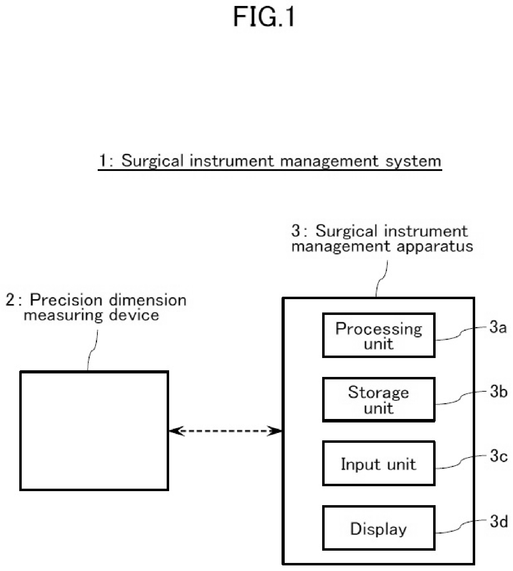

FIG. 1 is a block diagram showing an outline configuration of a surgical instrument management system to which a surgical instrument management method and apparatus according to an embodiment of the present invention are applied. As shown in FIG. 1, the surgical instrument management system 1 comprises: a precision dimension measuring device 2 that precisely measures dimensions of surgical instruments (not shown); and a surgical instrument management apparatus 3 that obtains the dimensions measured by the precision dimension measuring device 2 and manages the surgical instruments by individually identifying them based on the obtained dimensions.

The precision dimension measuring device 2 is a device that includes a table (not shown) and optically measures the dimensions of the surgical instruments placed on the table. Specifically, the precision dimension measuring device 2 measures the dimensions of predetermined specific portions of the surgical instruments (i.e., the portions designated by the surgical instrument management apparatus 3). For example, the precision dimension measuring device 2 captures images of the surgical instruments placed on the table, and determines the dimensions of the specific portions of the surgical instruments by analyzing the captured images. In addition, for example, the precision dimension measuring device 2 is capable of measuring the dimensions in micron or submicron units, and is configured such that its repeat measurement accuracy falls within a tolerance of several microns or submicrons.

The surgical instrument management apparatus 3 is configured by a general-purpose computer. The surgical instrument management apparatus 3 controls the precision dimension measuring device 2 by transmitting control signals to the precision dimension measuring device 2, in order to measure the dimensions of the predetermined specific portions for each of the plurality of surgical instruments. The surgical instrument management apparatus 3 also receives measurement signals corresponding to the dimensions of the specific portions measured by the precision dimension measuring device 2, and performs processing to individually identify and manage the surgical instruments. For example, the specific portions are designated in advance by a user, and may include portions that are characteristic for each of the plurality of surgical instruments (i.e., portions that are distinctive compared with other portions of the same surgical instrument), portions that are less susceptible to deterioration or aging, or portions that are easy to measure in terms of dimensions. Moreover, for the surgical instruments belonging to the same group or type, the same portion is used as the specific portion.

In the present embodiment, the surgical instrument management apparatus 3 causes the precision dimension measuring device 2 to measure the dimensions of the specific portions of each of the plurality of surgical instruments in advance, and the apparatus 3 preliminarily stores the dimensions of the specific portions measured for each of the plurality of surgical instruments (hereinafter referred to as "master registration" as appropriate). In this case, the surgical instrument management apparatus 3 uses numerical values (for example, numerical values in micron or submicron units) corresponding to the dimensions of the specific portions, as IDs, in order to identify and manage each of the plurality of surgical instruments. The embodiment is based on the idea that, when the plurality of surgical instruments are viewed in terms of their precisely measured dimensions, all of the surgical instruments can be distinguished-specifically, not only surgical instruments of different types, but even surgical instruments of exactly the same type can be distinguished. This is because individual differences appear when viewed through precisely measured dimensions.

After the above-described master registration has been completed, when the surgical instrument has actually been used, the surgical instrument management apparatus 3 causes the precision dimension measuring device 2 to measure the dimension of the specific portion of the surgical instrument, and the apparatus 3 compares the measured dimension with the dimension (corresponding to the numerical value of the ID) previously stored through the master registration, to individually identify the measured surgical instrument. At this time, as the management of the identified surgical instrument, the surgical instrument management apparatus 3 appropriately updates information relating to the surgical instrument, such as its usage history. The surgical instruments repeatedly go through the cycle of surgery, disinfection, cleaning, and sterilization. In a typical example, when the surgical instrument is returned to the sterilization-container after cleaning, the above individual identification and management of the surgical instrument are performed. In this case, the surgical instrument management apparatus 3 manages the surgical instruments in sterilization-container units including the plurality of surgical instruments.

More specifically, as shown in FIG. 1, the surgical instrument management apparatus 3 comprises: a processing unit 3a having one or more microprocessors serving as a central processing unit (CPU) for executing programs; a storage unit 3b, configured of a RAM (Random Access Memory), a ROM (Read Only Memory), or the like, for storing programs and data; an input unit 3c through which information is entered by a user via devices such as a mouse or a keyboard; and a display 3d for displaying various information.

In the present embodiment, the processing unit 3a executes programs for controlling the measurement of dimensions by the precision dimension measuring device 2, as well as programs for individually identifying and managing the surgical instruments based on the dimensions measured by the precision dimension measuring device 2. The processing unit 3a functions as the "obtaining unit," the "identifying unit," and the "managing unit" in the present invention. The storage unit 3b stores these programs and stores information relating to the plurality of surgical instruments in order to manage them. In particular, the storage unit 3b stores information such as a name of the surgical instrument, the measured dimension of the specific portion, a group name of the surgical instrument, a subcategory of the surgical instrument, a manufacturer name of the surgical instrument, a usage history of the surgical instrument (including histories of patient, cleaning, sterilization, storage), a service life of the surgical instrument, a repair history of the surgical instrument, and a purchase history of the surgical instrument, in association with the IDs (i.e., the numerical values of the dimensions as mentioned above) indicating each of the plurality of surgical instruments.

The group name of the surgical instrument corresponds to a relatively broad classification of the surgical instruments, such as forceps, scissors, tweezers, and needle holders. The subcategory of the surgical instrument corresponds to a further subdivision of these groups. As to the forceps, there are Kocher forceps, long Kocher forceps, mosquito Kocher forceps, Pean forceps, mosquito Pean forceps, baby mosquito Pean forceps, micro mosquito Pean forceps, Kelly forceps, long Kelly forceps, mosquito Kelly forceps, Mikulicz forceps, Magill forceps, bulldog forceps, Allis forceps, lymph node forceps, gastric forceps, intestinal forceps, and so on. The forceps may also be classified into hooked forceps and non- hooked forceps based on the presence or absence of hooks at the tips. As to the scissors, there are Cooper scissors, Mayo scissors, Metzenbaum scissors, ophthalmic scissors, and others, and they may further be classified into straight scissors with straight blade and curved scissors with curved blade. As to the tweezers, there are hooked tweezers and non-hooked tweezers, as well as instruments having different roles, such as Adson tweezers, McKando tweezers, DeBakey tweezers, and diamond tweezers whose tips are processed with the diamond coating. For the needle holders, there are Mathieu needle holders and Hegar needle holders. Furthermore, the subcategories of hooks are classified into muscle hooks, saddle hooks, liver hooks, single-hook retractors, multi-hook retractors, Steeple retractors.

### [Examples of Dimensions of Specific Portions]

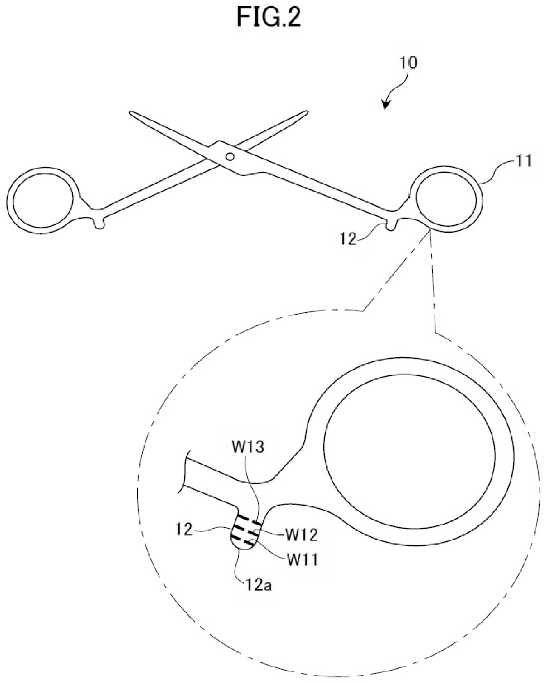

Next, with reference to FIGS. 2 to 7, specific examples of the dimensions of specific portions of the surgical instruments used in the present embodiment will be described.

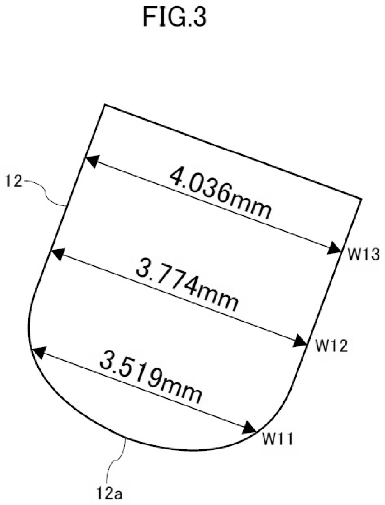

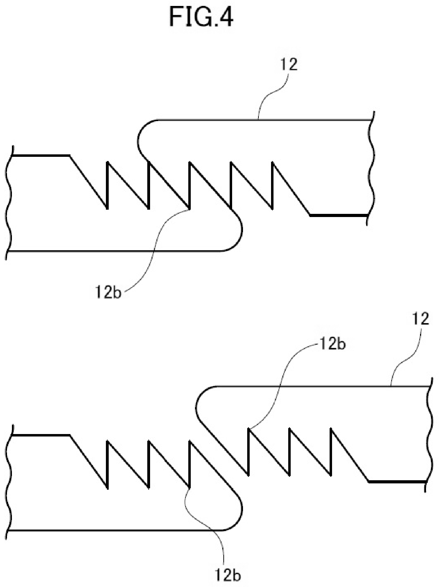

First, FIGS. 2 to 4 are explanatory diagrams showing the dimensions of specific portions of the forceps 10. Specifically, FIG. 2 shows an overall view of the forceps 10 in an open state and an enlarged view of the ratchet 12 of the forceps 10. FIG. 3 shows a further enlarged view of the ratchet 12 of the forceps 10, and FIG. 4 shows an enlarged view of the grooves 12b formed in the ratchet 12. In the present embodiment, the surgical instrument management apparatus 3 uses the ratchet 12, which is a protruding portion of the forceps 10, as the above-mentioned specific portion, in order to identify each of the plurality of forceps 10 based on the width of the ratchet 12.

Specifically, the surgical instrument management apparatus 3 uses the width of the portion located a predetermined distance from the tip 12a of the ratchet 12. In FIGS. 2 and 3, examples are shown of the width W11 at the position 1 mm from the tip 12a, the width W12 at the position 3 mm from the tip 12a, and the width W13 at the position 5 mm from the tip 12a. As an example, when these widths W11, W12 and W13 are measured using the precision dimension measuring device 2, they are 3.519 mm, 3.774 mm, and 4.036 mm, respectively. For instance, the precision dimension measuring device 2 specifies, from the captured image of the forceps 10, the ring portion 11 of the forceps 10 into which the user's fingers are inserted, and then specifies the ratchet 12 located near the ring portion and its tip 12a. Subsequently, the precision dimension measuring device 2 measures the widths (one or more of W11, W12 and W13) of the portions located the predetermined distances from the tip 12a.

The ratchet 12 of the forceps 10 has a plurality of grooves 12b, as shown in FIG. 4 (in FIG. 4, the upper diagram shows the ratchet 12 in a closed state, and the lower diagram shows the ratchet 12 in an open state). The grooves 12b are used to lock the forceps 10 in place at the position where a blood vessel is clamped. The measurement of the above-mentioned width of the ratchet 12 is performed on the surface opposite to the surface on which the grooves 12b are formed. Specifically, the precision dimension measuring device 2 performs the dimensional measurement while the surface of the ratchet 12 with the grooves 12b is facing downward and the surface without the grooves 12b is facing upward.

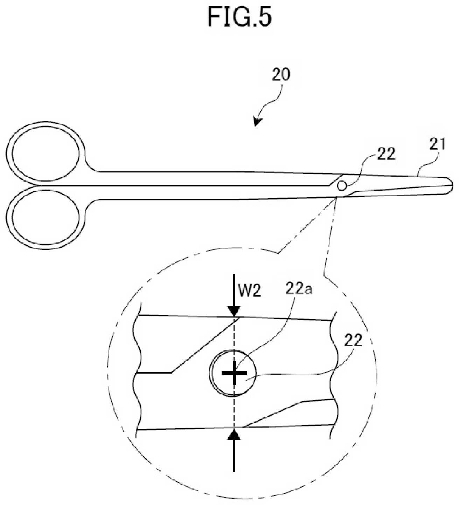

Next, FIG. 5 is an explanatory diagram showing the dimension of specific portion of the scissors 20. FIG. 5 shows an overall view of the scissors 20 in a closed state and an enlarged view of the pivot shaft 22 of the scissors 20. In the present embodiment, the surgical instrument management apparatus 3 uses the pivot shaft 22 of the scissors 20 as the above-mentioned specific portion, and identifies each of the plurality of scissors 20 based on the width W2 of the portion that passes through the center 22a of the pivot shaft 22. As an example, when the width W2 is measured using the precision dimension measuring device 2, the width W2 is 7.801 mm. For instance, the precision dimension measuring device 2 specifies, from the captured image of the scissors 20, the blade portion 21 of the scissors 20, and then specifies the pivot shaft 22 located near the blade portion 21 and its center 22a. Subsequently, the precision dimension measuring device 2 measures the width W2 of the portion passing through the center 22a. Instead of first specifying the blade portion 21 and then specifying the pivot shaft 22, the pivot shaft 22 may be specified directly.

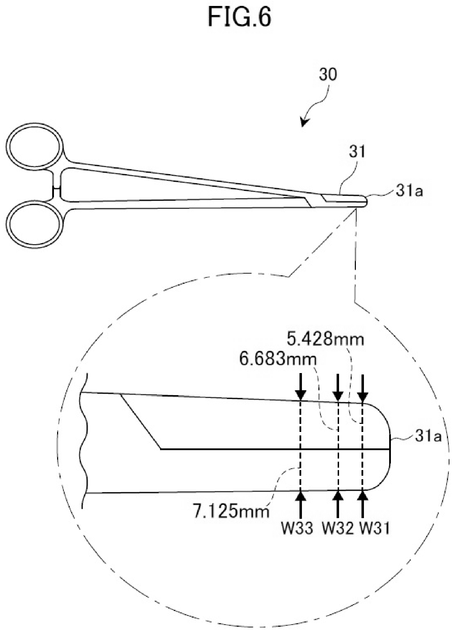

Next, FIG. 6 is an explanatory diagram showing the dimensions of specific portions of the needle holder 30. FIG. 6 shows an overall view of the needle holder 30 in a closed state and an enlarged view of the tip 31a of the gripping portion 31 of the needle holder 30. In the present embodiment, the surgical instrument management apparatus 3 uses the tip 31a located at the end of the gripping portion 31 as the above-mentioned specific portion, and identifies each of the plurality of needle holders 30 based on the width near the tip 31a. Specifically, the surgical instrument management apparatus 3 uses the width of the portion located a predetermined distance from the tip 31a of the gripping portion 31. In FIG. 6, examples are shown of the width W31 at the position 3 mm from the tip 31a, the width W32 at the position 5 mm from the tip 31a, and the width W33 at the position 10 mm from the tip 31a. As an example, when these widths W31, W32 and W33 are measured using the precision dimension measuring device 2, they are 5.428 mm, 6.683 mm, and 7.125 mm, respectively. For instance, the precision dimension measuring device 2 specifies, from the captured image of the needle holder 30, the gripping portion 31 of the needle holder 30, and then specifies the tip 31a of the gripping portion 31. Subsequently, the precision dimension measuring device 2 measures the widths (one or more of W31, W32 and W33) of the portions located the predetermined distances from the tip 31a.

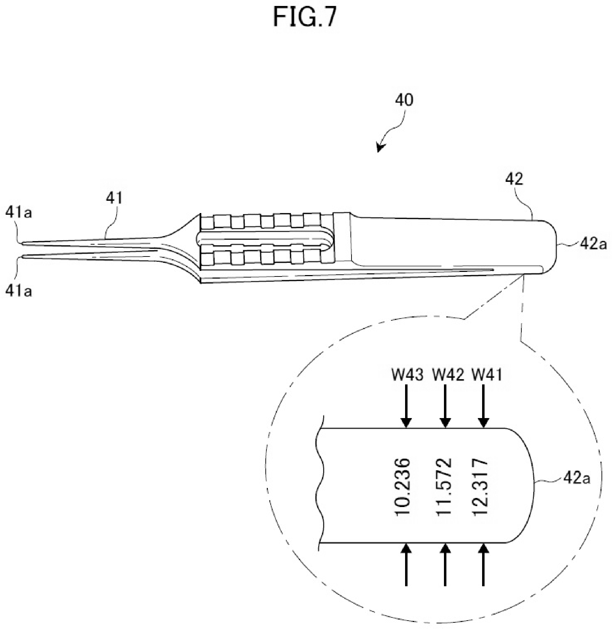

Next, FIG. 7 is an explanatory diagram showing the dimensions of specific portions of the tweezers 40. FIG. 7 shows an overall view of the tweezers 40 and an enlarged view of the rear end 42a of the connecting portion 42 of the tweezers 40. In the present embodiment, the surgical instrument management apparatus 3 uses, as the above-mentioned specific portion, not the tip 41a of the gripping portion 41 of the tweezers 40, but the rear end 42a of the connecting portion 42 where the two members are joined, and identifies each of the plurality of tweezers 40 based on the width near the rear end 42a. Specifically, the surgical instrument management apparatus 3 uses the width of the portion located a predetermined distance from the rear end 42a of the connecting portion 42. In FIG. 7, examples are shown of the width W41 at the position 3 mm from the rear end 42a, the width W42 at the position 5 mm from the rear end 42a, and the width W43 at the position 10 mm from the rear end 42a. As an example, when these widths W41, W42 and W43 are measured using the precision dimension measuring device 2, they are 12.317 mm, 11.572 mm and 10.236 mm, respectively. For instance, the precision dimension measuring device 2 specifies, from the captured image of the tweezers 40, the connecting portion 42 of the tweezers 40, and then specifies the rear end 42a of the connecting portion 42. Subsequently, the precision dimension measuring device 2 measures the widths (one or more of W41, W42 and W43) of the portions located the predetermined distances from the rear end 42a.

In the examples shown in FIGS. 2 to 7, the measurement range of the precision dimension measuring device 2 is narrow; that is, the dimensional measurement is performed not over the entire area of the surgical instruments, but only within a limited area, specifically a narrow region that includes the specific portion. As such, the measurement time by the precision dimension measuring device 2 can be reduced. Therefore, the precision dimension measuring device 2 is able to perform dimensional measurement on the plurality of surgical instruments in a relatively short time. As a result, by placing the plurality of surgical instruments on the table of the precision dimension measuring device 2 and measuring them in the same process, the dimensional measurement of the plurality of surgical instruments can be carried out efficiently.

Since the needle holder 30 also has the ratchet similar to the forceps 10, the width of the ratchet of the needle holder 30 may be used as the dimension of the specific portion. In addition, since the forceps 10 and the needle holder 30 also have the pivot shaft similar to the scissors 20, the width of the portion passing through the center of the pivot shaft of the forceps 10 or the needle holder 30 may be used as the dimension of the specific portion. Furthermore, as with the needle holder 30 and the tweezers 40, the width of an end portion (typically the tip) of the forceps 10 or the scissors 20 may also be used as the dimension of the specific portion.

The above examples illustrate commonly used instruments such as forceps 10, scissors 20, needle holders 30 and tweezers 40, which are referred to as common instruments. However, in addition to these common instruments, there exist several thousand other types of medical surgical instruments. As to other surgical instruments besides the common instruments, the dimensions of the specific portions can be defined to perform the individual identification and management based on the dimensions, similarly to the common instruments. In such a case, it is preferable to classify the several thousand types of the surgical instruments into the common instruments and other specialized instruments, to perform the measurement, identification and management of the surgical instruments. Although scalpels (scalpel blades) are disposable after surgery and therefore do not require the identification or management, scalpel holders are reusable and thus require identification and management.

### [Processing Flow]

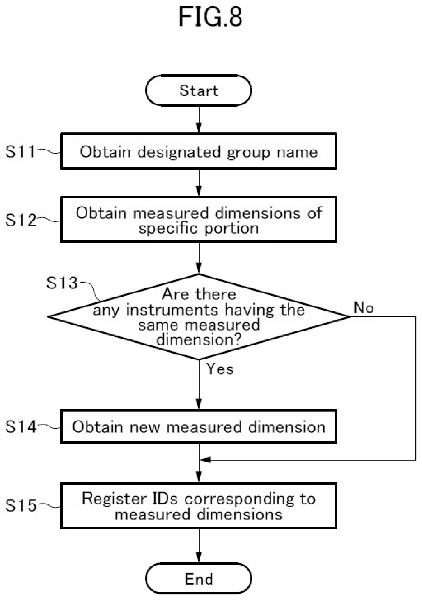

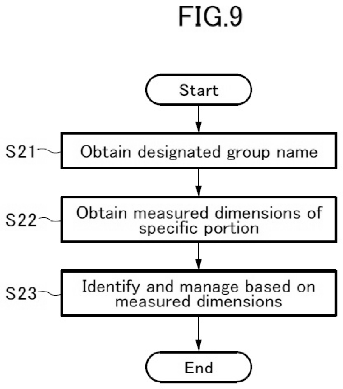

Next, with reference to FIGS. 8 and 9, the specific processing performed by the surgical instrument management apparatus 3 in the present embodiment will be described. FIG. 8 is a flowchart showing the master registration of the surgical instruments performed by the surgical instrument management apparatus 3 in the present embodiment, and FIG. 9 is a flowchart showing the individual identification and management of the surgical instruments performed by the surgical instrument management apparatus 3 in the present embodiment. These flows are implemented by the processing unit 3a of the surgical instrument management apparatus 3, which reads and executes the program stored in the storage unit 3b.

First, in the flow shown in FIG. 8, in Step S11, the surgical instrument management apparatus 3 obtains the group name of the plurality of surgical instruments that are set as the measurement targets of the precision dimension measuring device 2 (that is, the instruments placed on the table of the precision dimension measuring device 2), wherein the group name is designated by the user via the input unit 3c. Basically, the plurality of surgical instruments belong to the same group and have the same specific portion defined. As described above, in the present embodiment, the precision dimension measuring device 2 measures the dimensions of the specific portions of the plurality of surgical instruments in the same measuring process, and the surgical instrument management apparatus 3 obtains the dimensions of the plurality of specific portions in the same obtaining process.

As another example, instead of using the group name designated by the user, the group name to which the plurality of surgical instruments belong may be identified by analyzing the image captured by the precision dimension measuring device 2. As still another example, instead of or in addition to using the group name of the surgical instruments, the aforementioned subcategory of the surgical instruments may also be used.

Next, in Step S12, the surgical instrument management apparatus 3 identifies the specific portion that is predefined for the surgical instruments belonging to the group obtained in Step S11, and controls the precision dimension measuring device 2 to measure the dimensions of the identified specific portion. The surgical instrument management apparatus 3 then obtains the dimensions of the specific portion measured by the precision dimension measuring device 2. In this case, the surgical instrument management apparatus 3 obtains the dimensions of the specific portion of each of the plurality of surgical instruments that are measured by the precision dimension measuring device 2 in the same process.

Next, in Step S13, the surgical instrument management apparatus 3 determines whether any of the obtained dimensions of the plurality of specific portions are identical. When the plurality of measured dimensions fall within a predetermined tolerance (for example, ±5 microns), the surgical instrument management apparatus 3 determines that the dimensions of the specific portion are the same.

As a result of Step S13, when the surgical instrument management apparatus 3 determines that there are no instruments with identical measured dimensions (Step S13: No), it proceeds to Step S15. In Step S15, the apparatus 3 assigns, to each of the plurality of surgical instruments, the numerical value corresponding to the dimension of the specific portion (for example, the numerical value in micron or submicron units) as an ID, and manages these instruments based on the assigned IDs. In this case, the storage unit 3b in the surgical instrument management apparatus 3 stores information such as a name of the surgical instrument, the measured dimension of the specific portion, a group name of the surgical instrument, a subcategory of the surgical instrument, a manufacturer name of the surgical instrument, a usage history of the surgical instrument, a service life of the surgical instrument, a repair history of the surgical instrument, and a purchase history of the surgical instrument, in association with the ID assigned to each surgical instrument.

In contrast, when the surgical instrument management apparatus 3 determines that there are instruments with the identical measured dimensions (Step S13: Yes), the process proceeds to Step S14. As described above, the precision dimension measuring device 2 has repeatability accuracy within a tolerance of several microns or sub-microns, making the likelihood of identical measured dimensions extremely low. However, it is still possible for the surgical instruments manufactured by the same maker and belonging to the same production lot to have dimensions that match down to the micron or submicron level.

In step S14, the surgical instrument management apparatus 3 causes the precision dimension measuring device 2 to measure a dimension different from the initially measured specific portion for the plurality of surgical instruments whose dimensions of the specific portion were identical in the initial measurement, in order to obtain the newly measured dimension. In a typical example, the precision dimension measuring device 2 measures another dimension in the same specific portion. As an example, as shown in FIGS. 2 and 3, if the width W11 of forceps 10 was used initially, the width W12 or W12 may be newly used. After step S14, the surgical instrument management apparatus 3 performs the processing in step S15 in the same manner as described above. When the newly measured dimension is also identical among the instruments, an additional different dimension may be used. That is, a plurality of different dimensions may be measured until differing measurement results are obtained.

As another example, in step S14, instead of using a different dimension from the specific portion for the plurality of surgical instruments whose dimensions of the specific portion are identical, the specific portion of each surgical instrument may be trimmed (shaved) by several microns to several tens of microns so as to create the difference in the dimensions of the specific portion. The dimension of the specific portion after trimming (i.e., the dimension in which the difference has been created) may then be used. In this case, it is not necessary to bring machining equipment into the hospital; it is sufficient to trim the specific portion of the surgical instrument using a precision file or an abrasive material. It is also preferable to trim a portion of the surgical instrument that has minimal impact on its function.

Next, the flow shown in FIG. 9 will be explained. This flow is executed when the surgical instruments that have been disinfected and cleaned after surgery are returned to the sterilization-container. The flow is also executed for the set assembly which is returned to the sterilization-container, and the sterilization-container units.

First, in step S21, the surgical instrument management apparatus 3 obtains the group name of the plurality of surgical instruments that are set as the measurement targets of the precision dimension measuring device 2 (that is, the instruments placed on the table of the precision dimension measuring device 2), wherein the group name is designated by the user via the input unit 3c. Basically, the plurality of surgical instruments belong to the same group and have the same specific portion defined. As described above, in the present embodiment, the precision dimension measuring device 2 measures the dimensions of the specific portions of the plurality of surgical instruments in the same measuring process, and the surgical instrument management apparatus 3 obtains the dimensions of the plurality of specific portions in the same obtaining process.

As another example, instead of using the group name designated by the user, the group name to which the plurality of surgical instruments belong may be identified by analyzing the image captured by the precision dimension measuring device 2. As still another example, instead of or in addition to using the group name of the surgical instruments, the aforementioned subcategory of the surgical instruments may also be used.

Next, in Step S22, the surgical instrument management apparatus 3 identifies the specific portion that is predefined for the surgical instruments belonging to the group obtained in Step S21, and controls the precision dimension measuring device 2 to measure the dimensions of the identified specific portion. The surgical instrument management apparatus 3 then obtains the dimensions of the specific portion measured by the precision dimension measuring device 2. In this case, the surgical instrument management apparatus 3 obtains the dimensions of the specific portion of each of the plurality of surgical instruments that are measured by the precision dimension measuring device 2 in the same process.

Next, in step S23, the surgical instrument management apparatus 3 compares the dimensions measured by the precision dimension measuring device 2 with the dimensions previously stored through the master registration (which correspond to the numerical values of the IDs), and identifies each measured surgical instrument (that is, determines the ID). At this time, in order to manage the individually identified surgical instruments, the surgical instrument management apparatus 3 appropriately updates the stored information relating to the surgical instruments, such as usage history, cleaning history, or sterilization history.

It is preferable that the processes shown in FIGS. 8 and 9 are carried out in an environment maintained at a constant predetermined temperature, in order to suppress the effects of thermal expansion or thermal contraction of the surgical instruments (i.e., dimensional changes caused by temperature). In other words, it is desirable to perform these processes on the surgical instruments maintained at a constant predetermined temperature.

### [Operation and Effects]

As described above, in the present embodiment, the surgical instrument management apparatus 3 is configured to: obtain dimensions of specific portions that are predefined for a plurality of surgical instruments; to identify the surgical instruments based on the obtained dimensions of the specific portions; and manage the identified surgical instruments. The embodiment identifies the plurality of surgical instruments based on the dimensions of the predetermined specific portions in which individual differences appear among the plurality of surgical instruments (in other words, the dimensions that allow the instruments to be distinguished from one another). As a result, the identification and management of the surgical instruments can be performed easily, accurately, and at low cost. That is, according to the present embodiment, it is possible to identify and manage the surgical instruments without encountering the problems associated with the methods that use the two-dimensional codes or the RFID tags to individually identify and manage surgical instruments, as discussed in the section "Technical Problem."

Specifically, according to the present embodiment, since the surgical instruments are measured as they are, it is possible to accurately perform the individual identification and management even for the surgical instruments made of non-metal materials, such as ceramics or engineering plastics, for which the RFID tags cannot be attached, as well as for the small surgical instruments that have no space for attaching the RFID tag or the two-dimensional code.

According to the present embodiment, a laser marking machine for engraving the two-dimensional codes on the surgical instruments and a laser welding machine for attaching the RFID tags to the surgical instruments are not required, and it is unnecessary to bring such marking or welding machines into the hospital. In addition, since the labor required for such operations is eliminated, the total cost can be significantly reduced.

Furthermore, according to the present embodiment, manual operations are reduced, and variations in quality due to the skill level of personnel are eliminated. In particular, according to the present embodiment, the individual identification and management can be performed simply by placing the surgical instruments on the table of the precision dimension measuring device 2 and measuring them.

As described above, according to the present embodiment, it is possible to individually identify the surgical instruments in their original state, without processing the instruments to engrave the two-dimensional codes or attach the RFID tags. Moreover, all surgical instruments can be individually identified and managed regardless of their material, shape, or size.

It is preferable that the surgical instrument management apparatus 3 manages the surgical instruments on the sterilization-container units including the plurality of surgical instruments. In this case, the RFID tag or the two-dimensional code capable of withstanding the sterilization environment may be attached to the sterilization container, and a system may be constructed that enables identification of the types and quantities of surgical instruments contained within each sterilization container. In general, experienced personnel assemble the sets by removing surgical instruments from the storage shelves, and such set assembly relies heavily on the workers' experience and knowledge. Nevertheless, shortages, mix-ups, or excess instruments may occur during set assembly. By managing the surgical instruments as described above, unused instruments can be separated from used instruments after surgery, and unused instruments can be removed from the set, thereby streamlining the set contents. In addition, during surgical set assembly, the accuracy of the set assembly can be improved by recognizing the IDs of the surgical instruments and checking them off using the computer display. Furthermore, even staff members with limited expertise in the surgical instruments will be able to assemble the sets, thereby helping to alleviate labor shortages and reduce personnel costs. In the current situation where the surgical instruments are not properly managed, inventory control is insufficient. Specifically, it is not possible to ascertain the quantity, value, replenishment needs, repair status, number of uses, or service life of the surgical instruments within the hospital. However, by managing the surgical instruments as described above, the ordering operations can be automated, enabling labor savings. Ultimately, it becomes possible to eliminate the set-assembly process altogether and use the sterilization-containers themselves as surgical sets. Although the number of surgical instruments will increase compared with the current practice of reusing common surgical instruments across different set assemblies, the proposed management approach offers numerous significant advantages-such as reducing personnel costs through streamlined set contents and labor savings, improving the accuracy of set assembly, preventing excessive inventory through proper inventory control, and suppressing unnecessary and premature purchases of surgical instruments through appropriate service-life management.

### [Modification]

Next, modified examples of the above-described embodiment will be explained.

In the above embodiment, the identification is performed using the dimension (typically, the width) of the specific portion of the surgical instrument. As another example, the height of the surgical instrument may be obtained by three-dimensional measurement, and the surgical instrument may be identified using the height in addition to the dimension of the specific portion. For example, for the surgical instrument that includes the hook, the instrument may be identified based on the width and height of the hook.

As still another example, in addition to the dimension of the specific portion (and optionally the height), one or more of the following may further be used to identify the surgical instrument: a surface roughness of the surgical instrument, a surface gloss of the surgical instrument, a type of an element contained in a material constituting the surgical instrument, and a composition ratio of the element.

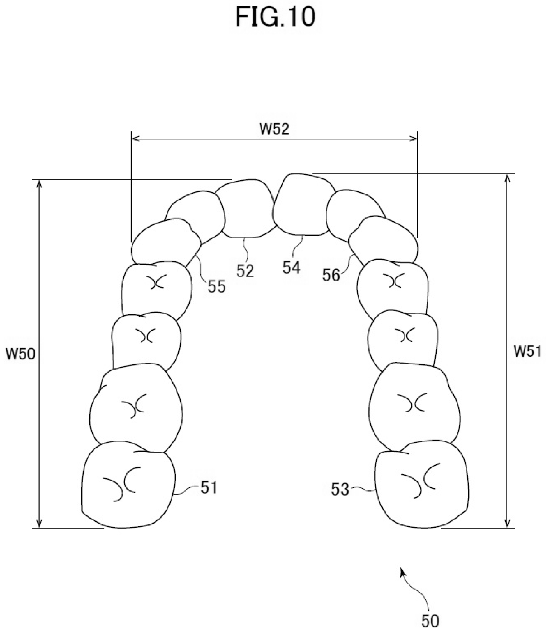

Although the above embodiment shows an example (i.e., a surgical instrument management method and apparatus) in which the present invention is applied to the surgical instruments, the present invention can be applied not only to the surgical instruments but also to orthodontic mouthpieces. That is, the surgical instrument management method and apparatus described above can be configured as an orthodontic mouthpiece management method and apparatus. The modified example will be explained below with reference to FIG. 10. FIG. 10 is a plan view of an orthodontic mouthpiece 50 used in the modified example. The orthodontic mouthpiece 50 is made of, for example, polyurethane and is formed to be transparent. In the modified example, the orthodontic mouthpiece management apparatus identifies a plurality of orthodontic mouthpieces 50 based on at least one of the following, which serve as the above-described specific portions: a longitudinal length W50 on one side of the orthodontic mouthpiece 50, a longitudinal length W51 on the other side, and a lateral length W52 perpendicular to these longitudinal directions. For example, the above precision dimension measuring device 2 identifies, from the captured image of the orthodontic mouthpiece 50, the molar portion 51 and the incisor portion 52 on one side, and then measures the length W50, which is the distance between these portions 51 and 52. Similarly, the precision dimension measuring device 2 identifies the molar portion 53 and the incisor portion 54 on the other side, and then measures the length W51, which is the distance between these portions 53 and 54. Further, the precision dimension measuring device 2 identifies a pair of tooth portions 55 and 56 arranged in the lateral direction, and then measures the length W52, which is the distance between these portions 55 and 56. Even for the same patient, the plurality of the orthodontic mouthpieces 50 are created depending on the stage of orthodontic treatment. By measuring at least one of the above lengths W50, W51 and W52, it is possible to accurately identify which orthodontic stage the particular orthodontic mouthpiece 50 was used in.

As described above, the embodiment discussed above is merely an example for explaining the present invention, and the present invention is not limited to this embodiment. The present invention may be implemented in various forms without departing from the spirit of the invention.

### LIST OF REFERENCE SIGNS

1: Surgical instrument management system
2: Precision dimension measuring device
3: Surgical instrument management apparatus
10: Forceps
12: Ratchet
20: Scissors
22: Pivot shaft
30: Needle holder
40: Tweezers
50: Orthodontic mouthpiece

## Claims

1. A surgical instrument management method for managing surgical instruments, comprising:
a step of obtaining dimensions of specific portions that are predefined for a plurality of surgical instruments;
a step of identifying the surgical instruments based on the obtained dimensions of the specific portions; and
a step of managing the identified surgical instruments.

2. The surgical instrument management method according to claim 1,
wherein the step of obtaining comprises, when the dimensions of the specific portion obtained from a plurality of the surgical instruments for which the same specific portion is defined are identical, newly obtaining one or more dimensions other than the dimensions of the specific portion, from the plurality of the surgical instruments, and
wherein the step of identifying comprises identifying the plurality of the surgical instruments based on the one or more dimensions.

3. The surgical instrument management method according to claim 1,
wherein the step of obtaining comprises, when the dimensions of the specific portion obtained from a plurality of the surgical instruments in which the same specific portion is defined are identical, newly obtaining the dimensions of the specific portion after trimming for one or more of the plurality of surgical instruments, and
wherein the step of identifying comprises identifying the plurality of surgical instruments based on the dimensions of the specific portion after trimming.

4. The surgical instrument management method according to claim 2 or 3, further comprising a step of determining that, when the dimensions of the specific portion obtained from the plurality of the surgical instruments fall within a predetermined tolerance, the dimensions of the specific portion are identical.

5. The surgical instrument management method according to claim 1,
wherein the step of obtaining comprises obtaining the dimensions of the specific portions of a plurality of the surgical instruments in the same step of obtaining, and
wherein the step of identifying comprises identifying the plurality of the surgical instruments based on the dimensions of the specific portions that have been obtained in the same step of obtaining.

6. The surgical instrument management method according to claim 1, further comprising a step of storing the dimensions of the specific portions obtained from a plurality of the surgical instruments in advance,
wherein the step of identifying comprises comparing the dimensions of the specific portions stored in the step of storing with the dimensions of the specific portions obtained in the step of obtaining after the step of storing, to identify the surgical instruments having the dimensions of the specific portions obtained in the step of obtaining.

7. The surgical instrument management method according to claim 1, wherein the dimension of the specific portion is a width of a protruding portion of the surgical instrument.

8. The surgical instrument management method according to claim 1, wherein the dimension of the specific portion is a width of an end portion of the surgical instrument.

9. The surgical instrument management method according to claim 1, wherein the dimension of the specific portion is a width of a portion of the surgical instrument that passes through a center of a pivot shaft of the surgical instrument.

10. The surgical instrument management method according to claim 1, wherein the step of identifying comprises identifying the surgical instruments based on numerical values in micron or submicron units of the dimensions of the specific portions.

11. The surgical instrument management method according to claim 1, wherein the step of identifying and the step of managing each comprise using numerical values corresponding to the dimensions of the specific portions, as IDs, in order to identify and manage the surgical instruments.

12. The surgical instrument management method according to claim 11, wherein the step of managing comprises managing, in association with the IDs, one or more of a name of the surgical instrument, a dimension of the specific portion of the surgical instrument, a group name of the surgical instrument, a subcategory of the surgical instrument, a manufacturer name of the surgical instrument, a usage history of the surgical instrument, a service life of the surgical instrument, a repair history of the surgical instrument, and a purchase history of the surgical instrument.

13. The surgical instrument management method according to claim 1, further comprising a step of obtaining respective heights of a plurality of the surgical instruments, and
wherein the step of identifying comprises identifying the surgical instruments based on both the obtained heights and the dimensions of the specific portions.

14. The surgical instrument management method according to claim 1, further comprising a step of obtaining one or more of a surface roughness of the surgical instrument, a surface gloss of the surgical instrument, a type of an element contained in a material constituting the surgical instrument, and a composition ratio of the element, and
wherein the step of identifying comprises identifying the surgical instruments based on one or more of the obtained surface roughness, surface gloss, type of the element, and composition ratio, in addition to the dimensions of the specific portions.

15. The surgical instrument management method according to claim 1, wherein the step of managing comprises managing the surgical instruments in sterilization-container units including a plurality of the surgical instruments.

16. A surgical instrument management apparatus for managing surgical instruments, comprising:
an obtaining unit configured to obtain dimensions of specific portions that are predefined for a plurality of surgical instruments;
an identifying unit configured to identify the surgical instruments based on the obtained dimensions of the specific portions; and
a managing unit configured to manage the identified surgical instruments.

17. An orthodontic mouthpiece management method, comprising:
a step of obtaining dimensions of specific portions that are predefined for a plurality of orthodontic mouthpieces;
a step of identifying the orthodontic mouthpieces based on the obtained dimensions of the specific portions; and
a step of managing the identified orthodontic mouthpieces.

18. An orthodontic mouthpiece management apparatus, comprising:
an obtaining unit configured to obtain dimensions of specific portions that are predefined for a plurality of orthodontic mouthpiece;
an identifying unit configured to identify the orthodontic mouthpiece based on the obtained dimensions of the specific portions; and
a managing unit configured to manage the identified orthodontic mouthpiece.
